# EUROPEAN PATENT APPLICATION

(11) **EP 4 059 574 A1**
(43) Date of publication of application: **21.09.2022**
(21) Application number: 20887716.7
(22) Date of filing: 14.10.2020
(51) Int. Cl.: A61Q 17/04, A61K 8/04, A61K 8/29, A61K 8/27, A61K 8/92

(54) **PHOTOPROTECTIVE COMPOSITIONS, PHOTOPROTECTIVE FORMULATIONS INCLUDING PHOTOPROTECTIVE COMPOSITIONS, PREPARATION METHODS AND USES THEREOF**

(30) Priority: 15.11.2019 BR 102019024166
(71) Applicant: Bioart Biocosméticos Ltda - ME, 88200-000 Tijucas-SC (BR); Universidade Federal de Santa Catarina, 88040-900 - Florianópolis-SC (BR)
(72) Inventor: ZONTA, Soraia Gomes, Florianópolis (BR); PEZZINI, Bianca Ramos, 88034-102 Florianópolis (BR); GOMES, Marina, 88117-35 Florianópolis (BR); CAON, Thiago, 88034-102 Florianopolis (BR)
(74) Representative: Pereira da Cruz, Joao
(86) International application number: PCT/BR2020/050414
(87) International publication number: WO 2021/092667

(57) **Abstract**

The present invention refers to compositions based on clays, oils and/or plant extracts with photoprotective and/or antioxidant activity and inorganic sunscreens; formulations including photoprotective compositions; methods of preparation and their uses. Problem to be solved: provide hybrid compositions between organic and inorganic photoprotective ingredients, which do not require the use of nanoparticle or synthetic chemical sunscreens, which provide adjustable degrees of SPF and FPUVA and that allow obtaining photoprotective formulations in different pharmaceutical or cosmetic forms.

Problem resolution: Photoprotective compositions containing at least one clay, at least one oil and/or plant extract with photoprotective and/or antioxidant activity, and at least one inorganic sunscreen are revealed; being said clay is present in the composition in a concentration of 0.1 to 85% by weight; being said oil and/or plant extract is present in the composition at a concentration of 0.2 to 80%, by weight; and being said inorganic sunscreen is present in the composition at a concentration of 0.5 to 75%, by weight. Still, a photoprotective formulation is revealed - as well as its method of preparation - including said photoprotective composition.

## Description

### FIELD OF THE INVENTION

The present invention refers to: photoprotective compositions based on clays, oils and/or plant extracts with photoprotective and/or antioxidant activity and inorganic sunscreens; photoprotective formulations including photoprotective compositions; methods of preparation and their uses. These compositions and formulations are suitable for pharmaceutical and cosmetic use, and can be used in products such as sunscreen and/or makeup, for example.

### BACKGROUND OF THE INVENTION

As is common knowledge, skin photoprotection is necessary to prevent sunburn, delay photoaging, decrease the chances of developing skin cancer, among other damage caused by ultraviolet (UV) radiation.

Research reveals that exposure to UVA radiation can lead to effects as undesirable as UVB radiation. In this sense, the products for sun protection should protect against UVA and UVB in a single formulation and present adequate values of SPF (Sun Protection Factor), FPUVA (UVA Protection Factor) and critical wavelength (nm).

There are basically two types of sunscreens used to confer photoprotection, that is, inorganic (physical) UV filters and organic (chemical) UV filters. In addition, many sunscreens absorb or reflect radiation only in a part of the UV spectrum, generally, and it is necessary to combine sunscreens to achieve a broad-spectrum UVA/UVB photoprotective effect.

Synthetic chemical sunscreens have a limited UV absorption spectrum; low photostability, which may result in toxic degradation products; in addition to the risk of photoallergic reaction and systemic absorption, which can cause endocrine disorders.

On the other hand, physical sunscreens are safer, have high photostability and do not cause contact dermatitis or photoallergic reactions. The particle size of inorganic filters can be reduced by increasing the level of photoprotection by forming more uniform films on the skin and increasing the ability to reflect radiation. However, nanoparticulate sunscreens can permeate the skin, be cytotoxic, cause oxidative stress, DNA damage, damage to the nervous system and cancer.

Thus, and in order to balance the advantages and disadvantages of inorganic and organic sunscreens, hybrid compositions, that is, that comprise both photoprotective organic agents and photoprotective inorganic agents have emerged, each with its particularity, such as those revealed documents WO2015189630 and WO2015193644.

In addition, other examples of solutions that somehow approach the present invention and therefore fit to be cited in this memorial are described in documents US20160303019 and US20120107253.

In an example, it is noteworthy that document US20120107253 describes sunscreen formulations, whose composition is specifically based on natural oceanic clay, as well as known inorganic and/or organic sunscreens, so as to synergistically strengthen protection against UV radiation. According to the inventors, other clays were tested and caused agglomeration problems, reducing the values of SPF and FPUVA, not presenting the synergistic effect with sunscreens, observed for the oceanic natural clay.

To maintain the transparency of the formulation after application to the skin, many commercial formulations are prepared by associating titanium dioxide and nanoparticulate zinc oxide. However, nano-scale compounds induce the formation of free radicals after exposure to solar radiation, leading to cellular damage (photomutagenicity). To work around this problem, US20120107253 provides non-nanoparticulate zinc oxide as inorganic sunscreen.

Many sunscreens, coating and dispersing agents employed in photoprotective products can be irritating, toxic and photosensitizing. In addition, a high amount of oil can be used in the formulation, possibly making it comedogenic and conferring unpleasant sensory attributes to the product.

Therefore, although the solutions described above are functional to the objectives for which they were feasible, there is still a gap in the state of the art regarding the provision of hybrid compositions between organic and inorganic photoprotective ingredients, which do not require the use of nanoparticulate or synthetic chemical sunscreens, which provide adjustable degrees of SPF and FPUVA and that allow obtaining photoprotective formulations, in different pharmaceutical or cosmetic forms, with the following advantages: suitable for all skin phototypes; with pleasant odor, dry touch, proper spreadability on the skin; no oily appearance and no intense bleaching on the skin; non-sticky, non-irritating and non-allergenic; with antioxidant action, also enabling the addition of effective active ingredients in the treatment of various skin conditions, for example, acne, inflammation and photoaging, giving additional benefits to the final products.

It is on the basis of this scenario that the invention in question arises.

### INVENTION GOALS

Thus, it is the fundamental objective of the invention in question to reveal photoprotective compositions suitable for the production of photoprotective formulations and multifunctional formulations (e.g., makeupwith photoprotection) for application on the skin.

It is also the objective of the present invention to describe photoprotective compositions that allow the production of photoprotective formulations with adjustable degrees of protection against solar radiation (from low protection to very high protection, i.e., minimum SPF of 6.0 and maximum of 99.9, with FPUVA of at least 1/3 of the FPS), in addition to a critical wavelength greater than 370 nm.

Still, one more goal of the present invention is to describe a method based on experimental design (*Design of Experiments* - DOE), which allows to define the necessary amounts of each constituent of the compositions of the invention, to obtain formulations with the desired values of SPF, FPUVA and critical wavelength, in addition to other parameters of photoprotective efficacy (e.g., UVA/UVB ratio).

### SUMMARY OF THE INVENTION

The above objectives are fully achieved by:

Photoprotective composition comprising at least one clay, at least one oil and/or plant extract with photoprotective and/or antioxidant activity, and at least one inorganic sunscreen.

Photoprotective composition comprising synergistically effective amounts of at least one clay, at least one vegetable oil and/or extract with photoprotective and/or antioxidant activity, and at least one inorganic sunscreen.

Photoprotective composition, wherein the at least one clay is present in the composition at a concentration of 0.1 to 85% by weight; the at least one vegetable oil and/or extract with photoprotective and/or antioxidant activity is present in the composition at a concentration of 0.2 to 80%, by weight; and the at least one inorganic sunscreen is present in the composition at a concentration of 0.5 to 75% by weight.

Photoprotective composition, wherein the at least one clay comprises one or more of the following compounds: silicon dioxide, aluminum oxide, iron oxide, potassium oxide, titanium dioxide, magnesium oxide, calcium oxide, phosphorus oxide, sodium oxide, zinc oxide, ferrous oxide, manganese oxide, lithium oxide, iron.

Photoprotective composition, wherein the at least one clay comprises one or more of the following compounds: silicon dioxide, aluminum oxide, iron oxide, titanium dioxide, iron.

Photoprotective composition, wherein the at least one clay comprises one or more of the following group: Tersil G, Tersil R, Tersil N, White Clay, Tersil CDR, Tersil CB, Tersil CP, Tersil COR, Tersil CBV, Tersil CGY, Cocoa Brown, Sparclay SW, Sparclay SR, Sparclay SBV, Sparclay SDR, Sparclay SOR, Sparclay SGY, Sparclay SP, Kaolin.

Photoprotective composition, wherein the at least one oil and/or plant extract comprises one or more compounds among: phenolic, nitrogenous, carotenoids, tocophosphols and ascorbic acid.

Photoprotective composition, wherein the at least one oil and/or plant extract comprises one or more among the following group: green tea extract, green coffee extract, calendula extract, yerba mate extract, ginger extract, rosemary essential oil, lemongrass essential oil, essential oil of grapefruit, sweet orange essential oil, lavender essential oil, pitanga essential oil, verbene essential oil, acai vegetable oil, karanja vegetable oil, rosehip vegetable oil.

Photoprotective composition, wherein the at least inorganic sunscreen comprises one or more among the following group: titanium dioxide, zinc oxide and hydroxyapatite.

Photoprotective composition, wherein the at least one inorganic sunscreen comprises one or more among the following group: Titanium Dioxide (And) Hydrated Silica (And) Jojoba Esters; Titanium Dioxide (And) Aluminum Hydroxide (And) Stearoyl Glutamic Acid; Hydroxyapatite; Titanium Dioxide (and) Ethyl Macadamiate (and) Silica (and) Alumina (and) Stearic Acid (and) Polyhydroxystearic Acid; Butyloctyl Salicylate, Titanium Dioxide (nano) [Titanium Dioxide] (40%), Triceteareth-4 Phosphate, Dimethicone Crosspolymer, Silica; Butyloctyl Salicylate, Titanium Dioxide (nano) [Titanium Dioxide] (23%), C12-15 Alkyl Benzoate, Ethylhexyl Methoxycrylene, Triceteareth-4 Phosphate, Dimethicone Crosspolymer, Silica; Zinc Oxide [Zinc Oxide] (60%), Butyloctyl Salicylate, Triceteareth-4 Phosphate, Triethoxycaprylylsilane; Zinc Oxide [Zinc Oxide] (58%), Butyloctyl Salicylate, Ethylhexyl Methoxycrylene, Triceteareth-4 Phosphate, Triethoxycaprylylsilane; Titanium Dioxide, Dimethicone; Zinc Oxide (And) Simmondsia Chinensis (Jojoba) Seed Oil (And) Polyhydroxystearic Acid (And) Jojoba Esters; Titanium Dioxide (And) Stearoyl Glutamic Acid; Titanium Dioxide (And) Alumina (And) Jojoba Esters; Zinc Oxide (nano); Zinc Oxide (and) Triethoxycaprylylsilane (nano); Zinc Oxide (and) Triethoxycaprylyllsilane; Zinc Oxide; Zinc Oxide (And) Stearoyl Glutamic Acid; Zinc Oxide (And) Jojoba Esters.

Photoprotective composition, wherein the at least one inorganic sunscreen comprises one or more among the following group: A15-TiO2-S-NJE10, A1K-TiO2-ASG3, Apalight, EMP50TEL, HallBrite^{®} EZ-FLO TDX, HallBrite^{®} EZ-FLO TDX Plus, HallBrite^{®} EZ-FLO ZDX, HallBrite^{®} EZ-FLO ZDX Plus, HallBrite^{®} T-97, JOP80MZCJ, TiO2-IR300-SG3, TTO-NJE8, Z-COTE^{®}, Z-COTE^{®} HP1, Z-COTE^{®} LSA, ZnO-C, ZNO-C-ASG3J, ZnO-C-NJE3.

Photoprotective formulation, wherein the formulation is an emulsion, dispersion, paste, powder or stick and comprises the photoprotective composition.

Multifunctional formulation, wherein the formulation is an emulsion, dispersion, paste, powder or stick and comprises the photoprotective composition.

Photoprotective formulation comprising the photoprotective composition, wherein the photoprotective formulation comprises: Zinc oxide (and) Simmondsia chinensis (jojoba) seed oil (and) polyhydroxystearic acid (and) jojoba esters in concentration of 9 to 21% by weight of the composition; Euterpe oleracea fruit oil (and) tocopherol in concentration of 4 to 8% by weight of composition; Titanium dioxide (and) ethyl macadamiate (and) silica (and) alumina (and) stearic acid (and) polyhydroxystearic acid at a concentration of 3 to 7% by weight of composition; Heptyl undecylenate in concentration of 2 to 4% by weight of composition; Ethylhexyl olivate in concentration of 1 to 3% by weight of composition; a first type of Kaolin in a concentration of 0.5 to 5% by weight of the composition; a second type of Kaolin in a concentration of 0.5 to 5% by weight of the composition; Tapioca starch in concentration of 0.5 to 3% by weight of the composition; propanediol in concentration of 4 to 8% by weight of composition; Cetearyl olivate (and) sorbitan olivate in concentration of 3 to 7% by weight of composition; Cetyl palmitate (and) sorbitan palmitate (and) sorbitan olivate in concentration of 1 to 5% by weight of composition; Olive oil peg-7 esters in concentration of 1 to 3% by weight of composition; Glyceryl caprylate in concentration of 0.5 to 1.5% by weight of composition; Cymbopogon citratus flower water in concentration of 18 to 22% by weight of the composition; Camellia sinensis leaf extract in a concentration of 0.5 to 1.5% by weight of the composition; Aqua in concentration sufficient for 100% by weight of composition.

Photoprotective formulation comprising the photoprotective composition, wherein the photoprotective formulation comprises: Tapioca starch in concentration of 0.5 to 3% by weight of the composition; Polyglyceryl-2-stearate, glyceryl stearate, stearyl alcohol in concentration of 6 to 12% by composition weight; Propanediol from 4 to 8% by weight of composition; Olive oil peg-7 esters in concentration of 1 to 3% by weight of composition; Glyceryl caprylate in concentration of 0.5 to 2% by weight of composition; Euterpe oleracea fruit oil (and) tocopherol in concentration of 3 to 7% by weight of composition; Heptyl undecylenate in concentration of 2 to 4% by weight of composition; Ethylhexyl olivate in concentration of 1 to 3% by weight of composition; Cymbopogon citratus flower water in concentration of 8 to 16% by weight of composition; A first type of Kaolin in a concentration of 0.5 to 5% by weight of the composition; A second type of Kaolin in a concentration of 0.5 to 5% by weight of the composition; Water (Aqua) (And) Calendula officinalis Flower Extract (And) Phospholipids (From Soybean Lecithin) (And) Tocopheryl Acetate in a concentration of 1 to 5% by weight of the composition; Camellia sinensis leaf extract in a concentration of 0.5 to 3% by weight of the composition; Zinc Oxide (And) Simmondsia Chinensis (Jojoba) Seed Oil (And) Polyhydroxystearic Acid (And) Jojoba Esters at a concentration of 5 to 30% by weight of composition; Titanium dioxide (and) ethyl macadamiate (and) silica (and) alumina (and) stearic acid (and) polyhydroxystearic acid concentration of 2 to 10% by weight of the composition; Aqua in concentration sufficient for 100% by weight of composition.

Photoprotective formulation comprising the photoprotective composition, wherein the photoprotective formulation comprises: Tapioca starch in concentration of 2% by weight of the composition; Polyglyceryl-2-stearate, glyceryl stearate, stearyl alcohol in concentration of 9% by weight of composition; Propanediol of 6% by weight of composition; Olive oil peg-7 esters in concentration of 2% by weight of composition; Glyceryl caprylate in concentration of 1% by weight of composition; Euterpe oleracea fruit oil (and) tocopherol in concentration of 5% by weight of composition; Heptyl undecylenate in concentration of 3% by weight of composition; Ethylhexyl olivate in concentration of 2% by weight of composition; Cymbopogon citratus flower water in concentration of 10% by weight of composition; A first type of Kaolin in concentration of 2% by weight of composition; A second type of Kaolin in concentration of 1% by weight of composition; Water (Aqua) (And) Calendula officinalis Flower Extract (And) Phospholipids (From Soybean Lecithin) (And) Tocopheryl Acetate in a concentration of 3% by weight of the composition; Camellia sinensis leaf extract in concentration of 1% by weight of composition; Zinc Oxide (And) Simmondsia Chinensis (Jojoba) Seed Oil (And) Polyhydroxystearic Acid (And) Jojoba Esters at a concentration of 11% by weight of composition; Titanium dioxide (and) ethyl macadamiate (and) silica (and) alumina (and) stearic acid (and) polyhydroxystearic acid concentration of 5% by weight of composition; Aqua in concentration of 37% by weight of the composition.

Method of preparation of photoprotective formulation comprising the steps of: determine the necessary amounts of each constituent of the photoprotective composition by experimental design; aqua heating, in reactor, up to 40°C; incorporate Tapioca starch, under agitation, thereby generating a first mixture; keep the shaking and heating of the first mixture up to 75°C; subject the first mixture to a high shear system for five minutes; prepare a second mixture containing:Polyglyceryl-2-Stearate, Glyceryl Stearate, Stearyl Alcohol; Propanediol; Olive Oil PEG-7 Esters; Glyceryl Caprylate; heat the second mixture until complete fusion, in reactor, the temperature between 70 to 75°C; incorporate the second mixture into the first mixture, gradually, under agitation, generating a third mixture; subject the third mixture to the high shear system for ten minutes; prepare a fourth mixture containing: Euterpe oleracea fruit oil (and) tocopherol; Undecylenate Heptyl; Ethylhexyl Olive Tree; heat the fourth mixture, in reactor, to a temperature of 75°C; incorporate the fourth mixture into the third mixture, gradually under agitation, generating a fifth mixture; submit the fifth mixture to the high shear system for five minutes; cool the fifth mixture, under agitation; wait a period of time; prepare a sixth mixture, in reactor, under agitation, dispersing, in Cymbopogon citratus flower water, the following: a first type of kaolin; a second type of kaolin; Water(Aqua) (And) Calendula officinalis Flower Extract (And) Phospholipids (From Soybean Lecithin) (And) Tocopheryl Acetate; Camellia sinensis leaf extract; incorporate the sixth mixture into the fifth mixture, under agitation, generating a seventh mixture; subject the seventh mixture to the high shear system for one minute; after, incorporate the zinc oxide (And) Simmondsia Chinensis (Jojoba) Seed Oil (And) Polyhydroxystearic Acid (And) Jojoba Esters into the seventh mixture, generating an eighth mixture; subject the eighth mixture to the high shear system for seven minutes; incorporate into the eighth mixture, gradually, Titanium dioxide (and) ethyl macadamiate (and) silica (and) alumina (and) stearic acid (and) polyhydroxystearic acid, generating a ninth mixture; subject the ninth mixture to the high shear system for thirteen minutes; and stir the ninth mixture for 30 minutes.

Use of photoprotective composition, for protecting from ultraviolet radiation and/or combating photoaging.

Use of photoprotective formulation, for protecting from ultraviolet radiation and/or combating photoaging.

Use of multifunctional formulation, for protecting from ultraviolet radiation and/or combating photoaging.

Use of photoprotective composition comprising other additives, in the treatment of acne and/or inflammation, and wherein the composition is an emulsion, dispersion, paste, powder or stick.

Use of multifunctional formulation comprising other additives, as makeup, and wherein the formulation is an emulsion, dispersion, paste, powder or stick.

### BRIEF DESCRIPTION OF THE DRAWINGS

In order to better define and clarify the content of this patent application, the following figures are presented:
Figure 1 illustrates a Pareto chart with the main effects of the factors (A: titanium dioxide; B: zinc oxide; C: acai vegetable oil; D: clay - Kaolin; E: clay - Sparclay^{®} SGY) and the interactions between them in the FPS response *in vitro*;
Figure 2 illustrates a Pareto chart with the main effects of the factors (A: titanium dioxide; B: zinc oxide; C: acai vegetable oil; D: clay - Kaolin; E: clay - Sparclay^{®} SGY) and the interactions between them in uvapf response₀;
Figure 3 illustrates a Pareto chart with the main effects of the factors (A: titanium dioxide; B: zinc oxide; C: acai vegetable oil; D: clay - Kaolin; E: clay - Sparclay^{®} SGY) and the interactions between them in the critical wavelength response (nm); and
Figure 4 illustrates a Pareto chart with the main effects of the factors (A: titanium dioxide; B: zinc oxide; C: acai vegetable oil; D: clay - Kaolin; E: clay - Sparclay^{®} SGY) and the interactions between them in the UVA/UVB ratio response.

### DETAILED DESCRIPTION OF THE INVENTION

The present invention refers to new and inventive compositions and photoprotective or multifunctional formulations and overcomes deficiencies of the state of the art.

Photoprotective formulations include any preparations intended to be exposed to the skin for the sole or primary purpose of protecting it against UVA and UVB radiation, while multifunctional formulations include preparations for application on skin whose protection against UV radiation is not the main purpose, but an additional benefit of the product (e.g. Make-up with photoprotection).

Focusing on the above-mentioned objectives, therefore, photoprotective compositions consisting of the association between: at least one clay (0.1 to 85%), at least one oil and/or plant extract with photoprotective and/or antioxidant activity (0.2 to 80%), and at least one inorganic sunscreen (0.5 to 75%), for example, titanium dioxide and/or zinc oxide.

The group of clays represents one of the types of constituents of the compositions and formulations of the present invention. Some examples of clays that can be used in the invention are presented in Table 1.

Another type of constituent of the compositions and formulations of the present invention is represented by the group of vegetable oils and extracts with photoprotective and/or antioxidant activity. Some examples of these constituents that can be used in the invention are presented in Table 2.

The group of inorganic sunscreens represents another type of constituent of the compositions and formulations of the present invention. Some examples of these constituents that can be used in the invention are presented in Table 3.

**Table 1 - Examples of clays that can be used in the compositions and formulations of the present invention.**

| **Name*** | **Colo ur** | **Main mine ral** | **Composition (%)** | | | | | | | | | **Properties** |
|---|---|---|---|---|---|---|---|---|---|---|---|---|
| | | | **SiO 2** | **Al₂ Or₃** | **Fe2 Or₃** | **K₂O r** | **Uncl e2** | **Mg O** | **Ca o** | **P₂ Or₅** | **In₂ Or** | |
| Tersil G | Green | Zn | 65.0 0 | 18.0 0 | 4.20 | 1.20 | 0.8 5 | 0.7 5 | 0.7 5 | 0.1 5 | - | Oil reducer of the skin; matte effect; improves the stability of emulsions; improves the sensory of gels and emulsions. |
| Tersil R | Red | Fe | 60.0 0 | 21.5 0 | 6.50 | 1.15 | 1.7 0 | ≤0. 25 | ≤0. 15 | ≤0. 15 | ≤0. 05 | Antioxidant; anti-aging; oil reducer of the skin; matte effect; improves the stability of emulsions; improves the sensory of gels and emulsions. |
| Tersil N | Grey | Cr | 52.0 0 | 31.5 0 | 2.20 | 1.00 | 1.5 0 | 0.2 5 | ≤0. 15 | ≤0. 15 | ≤0. 15 | Antioxidant; anti-aging; oil reducer of the skin; matte effect; improves the stability of emulsions; improves the sensory of gels and emulsions. |
| White Clay | White | Mo | 58.0 0 | 25.0 0 | 0.85 | 0.75 | 0.4 5 | 0.2 5 | ≤0. 05 | ≤0. 15 | ≤0. 05 | Antioxidant; reducing the oil of the skin; matte effect; improves the stability of emulsions; improves the sensory of gels and emulsions. |
| Tersil CDR | Red dark | Fe | 40.0 0 | 35.0 0 | 11.0 0 | ≤0.2 5 | 1.5 0 | ≤0. 25 | ≤0. 05 | ≤0. 10 | ≤0. 05 | Antioxidant; anti-aging; oil reducer of the skin; matte effect; improves the stability of emulsions; improves the sensory of gels and emulsions. |
| Tersil CB | Black | Ca | 58.0 0 | 31.5 7 | 1.25 | 0.21 | 2.2 1 | 0.0 6 | 0.1 0 | 0.0 8 | 0.11 | Antioxidant; oil reducer of the skin; matte effect; improves the stability of emulsions; improves the sensory of gels and emulsions. |
| Tersil CP | Purpl e | Mg | 54.5 0 | 21.1 0 | 7.45 | 5.00 | 1.2 5 | 1.6 5 | ≤0. 05 | ≤0. 05 | ≤0. 15 | Antioxidant; oil reducer of the skin; matte effect; improves the stability of emulsions; improves the sensory of gels and emulsions. |
| Tersil COLO UR | Rosy | Fe | 55.0 0 | 30.0 0 | 3.50 | 0.85 | 1.5 0 | ≤0. 20 | ≤0. 15 | ≤0. 15 | ≤0. 15 | Antioxidant; anti-aging; oil reducer of the skin; matte effect; improves the stability of emulsions; improves the sensory of gels and emulsions. |
| Tersil CBV | Crea m | Mn | 54.5 0 | 30.5 0 | 1.50 | 0.48 | 1.6 0 | 0.2 6 | ≤0. 05 | ≤0. 15 | ≤0. 15 | Antioxidant; matte effect; improves the stability of emulsions; improves the sensory of gels and emulsions. |
| Tersil CGY | Yello w gold | Cr and Fe | 47.0 0 | 32.0 0 | 6.00 | 0.40 | 1.7 0 | ≤0. 35 | ≤0. 05 | ≤0. 15 | ≤0. 05 | Antioxidant; anti-aging; reducer oil of the skin; matte effect; improves the stability of emulsions; improves the sensory of gels and emulsions |
| Cocoa Brown | Brow n | P | 60.0 0 | 20.0 0 | 7.25 | 4.50 | 0.7 0 | 0.7 5 | 0.0 2 | 0.1 0 | 0.12 | Antioxidant; matte effect; improves the stability of emulsions; improves the sensory of gels and emulsions. |
| Sparcla y SW | White / Crea m | Mo | 58.0 0 | 25.0 0 | 0.85 | 0.75 | 0.4 5 | 0.2 5 | ≤0. 05 | ≤0. 15 | ≤0. 05 | Antioxidant; matte effect; improves the stability of emulsions; improves the sensory of gels and emulsions. |
| Sparcla y MR | Red | Fe | 60.0 0 | 21.5 0 | 6.50 | 1.15 | 1.7 0 | ≤0. 25 | ≤0. 15 | ≤0. 15 | ≤0. 05 | Antioxidant; anti-aging; matte effect; improves the stability of emulsions; improves the sensory of gels and emulsions. |
| Sparcla y SBV | Beige | Mn | 54.5 0 | 31.5 0 | 1.43 | 0.46 | 1.6 5 | 0.3 2 | ≤0. 05 | ≤0. 15 | ≤0. 15 | Antioxidant; matte effect; improves the stability of emulsions; improves the sensory of gels and emulsions. |
| Sparcla y SDR | Red dark | Fe | 40.0 0 | 35.0 0 | 11.0 0 | ≤0.2 5 | 1.5 0 | ≤0. 25 | ≤0. 05 | ≤0. 10 | ≤0. 05 | Antioxidant; anti-aging; reducing the oil of the skin; matte effect; improves the stability of emulsions; improves the sensory of gels and emulsions. |
| Sparcla y SER | Rosy | Fe | 55.0 0 | 30.0 0 | 3.50 | 0.85 | 1.5 0 | ≤0. 20 | ≤0. 15 | ≤0. 15 | ≤0. 15 | Antioxidant; anti-aging; oil reducer of the skin; matte effect; improves the stability of emulsions; improves the sensory of gels and emulsions. |
| Sparcla y Sgy | Yello w gold | Cr and Fe | 47.0 0 | 32.0 0 | 6.00 | 0.40 | 1.7 0 | ≤0. 35 | ≤0. 05 | ≤0. 15 | ≤0. 05 | Antioxidant; anti-aging; oil reducer of the skin; matte effect; improves the stability of emulsions; improves the sensory of gels and emulsions. |
| Sparcla v SP | Purpl e | Mg | 54.5 0 | 21.1 0 | 7.45 | 5.00 | 1.2 5 | 1.6 5 | ≤0. 05 | ≤0. 05 | ≤0. 15 | Antioxidant; anti-aging; oil reducer of the skin; matte effect; improves the stability of emulsions; improves the sensory of gels and emulsions. |
| Kaolin | White / Beige | - | 48.0 0 | 39.0 0 | - | 0.75 | 0.4 5 | 0.2 5 | ≤0. 05 | ≤0. 15 | ≤0. 05 | Oil reducer of the skin; matte effect; improves the stability of emulsions; improves the sensory of gels and emulsions. |
| ^{∗}Intemational Nomenclature of Cosmetic Ingredients - INCI: Kaolin; origin of the European Valley - SC and Tijucas do Sul - PR. | | | | | | | | | | | | |
| SiO₂ = Silicon dioxide; Al₂Or₃ = Aluminum oxide; Fe₂Or₃ = Iron oxide; K₂O = Potassium oxide; Uncle₂ = Titanium dioxide; | | | | | | | | | | | | |
| MgO =Magnesium oxide; CaO = Calcium oxide; P₂Or₅ = Phosphorus oxide; In₂O = Sodium oxide. | | | | | | | | | | | | |

**Table 2 - Examples of vegetable oils and extracts with photoprotective and/or antioxidant activity that can be used in compositions and formulations of invention.**

| **Name** | **International Nomenclature of Cosmetic Ingredients - INCI** | **FPS determined by Mansur method** | **Antioxidant activity determined by the DPPH method (%FRS = *free radical scavenging*)** |
|---|---|---|---|
| Aromatic extract of green tea (*Camellia Sinensis*) | Camellia sinensis leaf extract | 0.79 | 5.53 |
| Green tea glycolic extract (*Camellia Sinensis*) | Camellia sinensis leaf extract | 0.05 | 10.84 |
| Oily green coffee extract (*Arabica Coffea*) | Coffea arabica seed oil, Zea mays oil | 0.21 | 13.92 |
| Oily marigold extract (*Calendula officinalis*) | Calendula officinalis flower oil, Bixa orellana seed oil, Zea mays oil, tocopherol | 0.13 | 11.58 |
| Oily yerba mate extract (*Ilex Paraguariensis* St. Hilarie) | Ilex paraguariensis leaf oil, Zea mays oil | 0.17 | 14.32 |
| Oily ginger extract (*Zingiber officinale*) | Zingiber officinale root oil, Helianthus annuus seed oil | 0.12 | 18.63 |
| Rosemary essential oil (Officinalis Rosmarinus) | Rosmarinus leaf oil officinalis | 0.22 | 2.67 |
| Lemongrass essential oil (Citratus cymbopogon) | Cymbopogon Citratus Leaf Oil | 0.40 | 40.76 |
| Essential oil from grapefruit (Citrus Grandis) | Citrus grandis seed oil | 1.05 | 3.63 |
| Sweet orange essential oil (Citrus Dulcis Aurantium) | Citrus aurantium dulcis oil | 0.61 | 1.65 |
| Lavender essential oil (Lavandula angustifolia) | Lavandula angustifolia oil | 0.28 | 4.49 |
| Pitanga essential oil (Eugenia Uniflora) | Eugenia Uniflora Leaf Oil | 1.02 | 9.16 |
| Verdee essential oil (Lippia Alba) | Lippia alba leaf/stem oil | 3.45 | 2.65 |
| Acai vegetable oil (Euterpe Olerace) | Euterpe oleracea fruit oil (and) tocopherol | 0.45 | 29.75 |
| Karanja vegetable oil (Pongamia glabra) | Pongamia glabra seed oil | 7.02 | 5.82 |
| Vegetable oil rosehip (Canine rose) | Rose canine fruit oil | 0.06 | 12.67 |

**Table 3 - Examples of inorganic sunscreens that can be used in compositions and formulations of invention.**

| **Name** | **International Nomenclature of Cosmetic Ingredients - INCI** |
|---|---|
| A15-TiO2-S-NJE10 | Titanium Dioxide (And) Hydrated Silica (And) Jojoba Esters |
| A1K-TiO2-ASG3 | Titanium Dioxide (And) Aluminum Hydroxide (And) Stearoyl Glutamic Acid |
| Apalight | Hydroxyapatite |
| EMP50TEL | Titanium Dioxide (and) Ethyl Macadamiate (and) Silica (and) Alumina (and) Stearic Acid (and) Polyhydroxystearic Acid |
| HallBrite^{®} EZ-FLO ZDX | Zinc Oxide [Zinc Oxide] (60%), Butyloctyl Sallate, Triceteareth-4 Phosphate, Triethoxycaprylylsilane |
| HallBrite^{®} EZ-FLO ZDX Plus | Zinc Oxide [Zinc Oxide] (58%), Butyloctyl Salicylate, Ethylhexyl Methoxycrylene, Triceteareth-4 Phosphate, Triethoxycaprylylsilane |
| HallBrite^{®} T-97 | Titanium Dioxide, Dimethicone |
| JOP80MZCJ | Zinc Oxide (And) Simmondsia Chinensis (Jojoba) Seed Oil (And) Polyhydroxystearic Acid (And) Jojoba Esters |
| Ti02-IR300-ASG3 | Titanium Dioxide (And) Stearoyl Glutamic Acid |
| TTO-NJE8 | Titanium Dioxide (And) Alumina (And) Jojoba Esters |
| Z-COTE^{®} LSA | Zinc Oxide (and) Triethoxycaprylylsilane |
| ZnO-C | Zinc Oxide |
| ZNO-C-ASG3J | Zinc Oxide (And) Stearoyl Glutamic Acid |
| ZnO-C-NJE3 | Zinc Oxide (And) Jojoba Esters |

In order to outline the compositions and photoprotective formulations of the present invention, a method based on an experimental design was developed (*Design of Experiments* - *DOE*).

The experimental design (DOE) is a statistical tool that allows the evaluation, in a rational, controlled and reproducible way, the main effects of pre-established factors (independent variables), and the effects of interactions between them, on the responses (results or dependent variables) of interest.

In other words, the DOE allows defining the necessary amounts of each constituent of the compositions of the invention, to obtain formulations with the desired values of SPF, FPUVA and critical wavelength, in addition to other parameters of photoprotective efficacy (e.g., UVA/UVB ratio).

In an example of the present invention, a fractional factorial experimental design 2⁵⁻¹, with four central points (Table 4), was elaborated in the Design-Expert software^{®} (Stat-Ease), from the variation of the levels (concentrations) of five factors (constituents of the compositions of the invention): two clays (Kaolin and Sparclay^{®} SGY), a vegetable oil with photoprotective and antioxidant activities (acai oil) and two inorganic sunscreens (dispersions of titanium dioxide and zinc oxide), generating 20 experiments. These constituents were incorporated on an emulsified cosmetic basis according to the pre-established concentrations, and the resulting formulations were submitted to photoprotective efficacy analysis *in vitro* (answers: FPS *in vitro,* UVAPF₀, UVA/UVB ratio and critical wavelength), in a diffuse reflectance spectrophotometer with integrative sphere, model UV2000S (Labsphere^{®}). The samples were weighed and evenly applied to polymethylmethacryhomee plates. The samples in the plates were dried at room temperature under light before the analysis. The analyses were performed in triplicate and nine different points per plate were measured for each sample. The results obtained are presented in Table 4.

The responses of photoprotective efficacy obtained (Table 4) were analyzed in the Design-Expert software^{®} (Stat-Ease). All the effects of the factors and their interactions were evaluated by Variance Analysis (ANOVA) to determine the statistical relevance of each term. Considering the statistically significant terms (p<0.05), a mathematical model was established for each response, by linear regression, based on the best fit (*lack-of-fit* not significant - p>0.05). All models presented adequate accuracy (> 4). R values²> 0.96, R² adjusted > 0.93 and R²>.86 were obtained, showing the robustness of the mathematical models obtained.

**Table 4 - Fractional factorial design (DOE) 2⁵⁻¹, with four central points, elaborated for the development of photoprotective formulations from compositions of the invention, and corresponding photoprotective efficacy responses.**

| **Code** | **FACTORS** | | | | | **PHOTOPROTECTIVE EFFICACY RESPONSES** | | | |
|---|---|---|---|---|---|---|---|---|---|
| | **A** | **B** | **C** | **D** | **E** | **FPS *in vitro*** | **UVAPF 0** | **Critical wavelength (nm)** | **UVA/UVB ratio** |
| 1 | -1.0 | -1.0 | -1.0 | -1.0 | 1.0 | 15 | 9 | 379 | 0,753 |
| 2 | 1.0 | -1.0 | -1.0 | -1.0 | -1.0 | 20 | 9 | 378 | 0,701 |
| 3 | -1.0 | 1.0 | -1.0 | -1.0 | -1.0 | 29 | 21 | 380 | 0,818 |
| 4 | 1.0 | 1.0 | -1.0 | -1.0 | 1.0 | 91 | 40 | 379 | 0,770 |
| 5 | -1.0 | -1.0 | 1.0 | -1.0 | -1.0 | 16 | 8 | 379 | 0,761 |
| 6 | 1.0 | -1.0 | 1.0 | -1.0 | 1.0 | 52 | 18 | 379 | 0,702 |
| 7 | -1.0 | 1.0 | 1.0 | -1.0 | 1.0 | 46 | 26 | 380 | 0,824 |
| 8 | 1.0 | 1.0 | 1.0 | -1.0 | -1.0 | 69 | 30 | 379 | 0,774 |
| 9 | -1.0 | -1.0 | -1.0 | 1.0 | -1.0 | 13 | 8 | 379 | 0,753 |
| 10 | 1.0 | -1.0 | -1.0 | 1.0 | 1.0 | 43 | 16 | 379 | 0,726 |
| 11 | -1.0 | 1.0 | -1.0 | 1.0 | 1.0 | 56 | 34 | 380 | 0,826 |
| 12 | 1.0 | 1.0 | -1.0 | 1.0 | -1.0 | 91 | 38 | 379 | 0,786 |
| 13 | -1.0 | -1.0 | 1.0 | 1.0 | 1.0 | 17 | 10 | 379 | 0,753 |
| 14 | 1.0 | -1.0 | 1.0 | 1.0 | -1.0 | 60 | 21 | 378 | 0,725 |
| 15 | -1.0 | 1.0 | 1.0 | 1.0 | -1.0 | 82 | 41 | 379 | 0,788 |
| 16 | 1.0 | 1.0 | 1.0 | 1.0 | 1.0 | 100 | 34 | 379 | 0,786 |
| 17 | 0.0 | 0.0 | 0.0 | 0.0 | 0.0 | 38 | 19 | 379 | 0,769 |
| 18 | 0.0 | 0.0 | 0.0 | 0.0 | 0.0 | 38 | 22 | 379 | 0,773 |
| 19 | 0.0 | 0.0 | 0.0 | 0.0 | 0.0 | 39 | 20 | 379 | 0,772 |
| 20 | 0.0 | 0.0 | 0.0 | 0.0 | 0.0 | 38 | 19 | 379 | 0,772 |
| - Code: Experiment/Formulation | | | | | | | | | |
| - Factors: A - titanium dioxide; B - zinc oxide; C - acai oil; D - Kaolin; E: Sparclay^{®} Sgy. | | | | | | | | | |
| - The values -1.0; 0.0; and 1.0 are codes and indicate the lower, intermediate and upper levels of the factor, respectively. | | | | | | | | | |

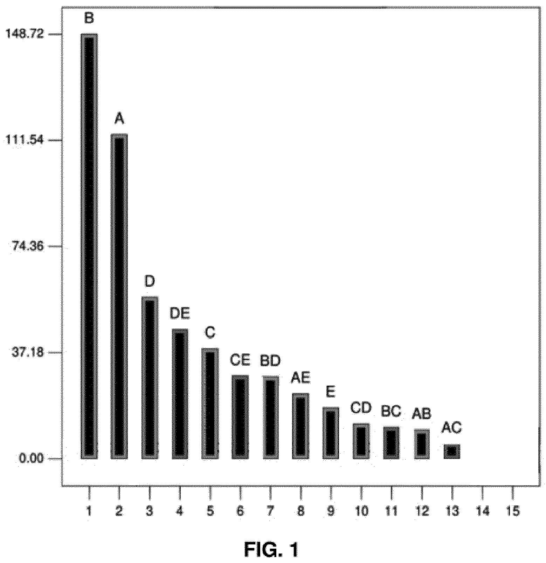

The data in Table 5 and Pareto's graphs in Figures 1, 2, 3 and 4 show that the responses of photoprotective efficacy of the invention formulations are influenced, in different magnitudes, by the constituents of the invention compositions (factors) and by the interactions that occur between them. These interactions are not obvious or expected and confer unique photoprotective properties to the compositions and formulations of the invention.

**Table 5 - Percentage (%) contribution of the main effects of the factors and interactions between them to the responses of photoprotective efficacy, in the fractional factorial experimental design (DOE) 2⁵⁻¹, with four central points.**

| **% CONTRIBUTION TO THE RESPONSE OF PROTECTIVE EFFECTIVENESS** | | | | |
|---|---|---|---|---|
| | **FPS *in vitro*** | **UVAPF₀** | **Critical wavelength (nm)** | **UVA/UVB ratio** |
| **MAIN:** | | | | |
| Titanium dioxide (A) | +28.00 | +6.67 | -37.63 | -25.31 |
| Zinc oxide (B) | +47.99 | +75.49 | +27.65 | +66.43 |
| Acai vegetable oil (C) | +3.23 | +0.59 | -0.77 | -0.091 |
| Kaolin (D) | +6.95 | +4.68 | -0.77 | +0.44 |
| Sparclay^{®} SGY (E) | +0.69 | +0.31 | +12.29 | +0.29 |
| INTERACTIONS: | | | | |
| AB | +0.22 | - | - | - |
| AC | -0.06 | - | - | +0.21 |
| AD | - | - | +6.91 | +3.36 |
| AE | +1.11 | - | - | -0.40 |
| BC | -0.26 | - | - | -0.31 |
| COMICS | +1.78 | - | -3.07 | -0.44 |
| BE | - | - | - | +0.60 |
| CD | +0.32 | - | -6.91 | -0.90 |
| EC | -1.82 | -3.68 | - | - |
| FROM | -4.43 | -3.96 | - | +0.50 |

Figure 1 illustrates a Pareto chart with the main effects of the factors (A: titanium dioxide; B: zinc oxide; C: acai vegetable oil; D: clay - Kaolin; E: clay - Sparclay^{®} SGY) and the interactions between them in the FPS response *in vitro.*

As shown in the graph, in Figure 1, factors B, A, D, C, E - and the BD, AE, CD and AB interactions - identified by orange color, have positive effects on the SPF response *in vitro,* while the DE, CE, BC and CA interactions, identified by blue color, have negative effects on this response. The effects on columns 14 and 15 are not significant.

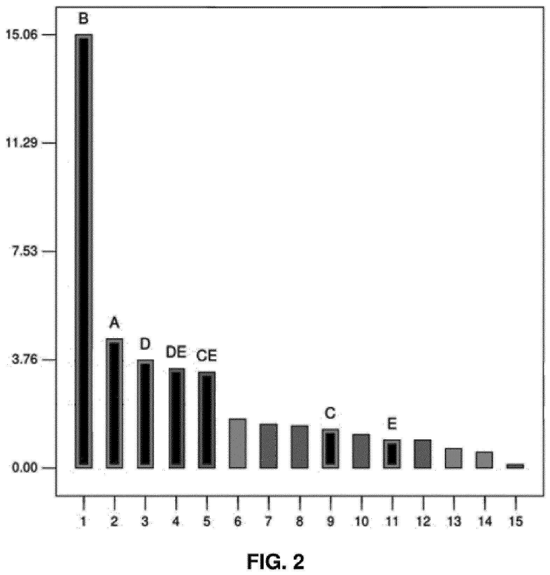

Figure 2 illustrates a Pareto chart with the main effects of the factors (A: titanium dioxide; B: zinc oxide; C: acai vegetable oil; D: clay - Kaolin; E: clay - Sparclay^{®} SGY) and the interactions between them in uvapf response₀.

As shown in the graph, in Figure 2, factors B, A, D, C and E, identified by orange, have positive effects on uvapf response₀, while THE and EC interactions, identified by blue, have negative effects on this response. The effects on columns 6, 7, 8, 10, 12, 13, 14 and 15 are not significant.

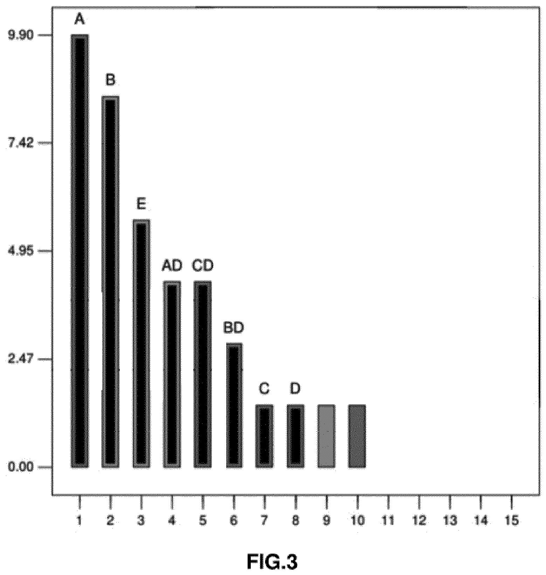

Figure 3 illustrates a Pareto chart with the main effects of the factors (A: titanium dioxide; B: zinc oxide; C: acai vegetable oil; D: clay - Kaolin; E: clay - Sparclay^{®} sgy) and the interactions between them in the critical wavelength response (nm).

As shown in the graph, in Figure 3, factors B and E - and interaction AD - identified by orange, have positive effects on the critical wavelength response (nm), while factors A, C and D - and CD and BD interactions - identified by blue color, have negative effects on this response. The effects on columns 9 and 10 are not significant.

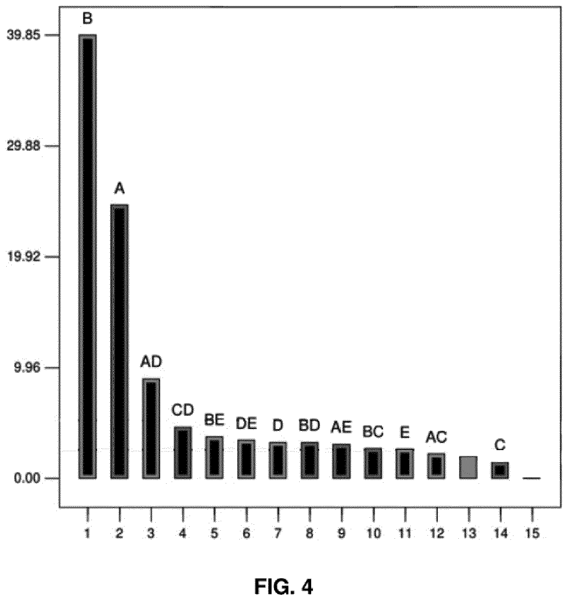

Figure 4 illustrates a Pareto chart with the main effects of the factors (A: titanium dioxide; B: zinc oxide; C: acai vegetable oil; D: clay - Kaolin; E: clay - Sparclay^{®} sgy) and interactions between them in the UVA/UVB ratio response.

As shown in the graph, in Figure 4, factors B, D and E - and the interactions AD, BE, DE and CA - identified by orange, have positive effects on the UVA/UVB ratio response, while factors A and C - and cd, BD, AE and BC interactions - identified by blue color, have negative effects on this response. The effects on columns 13 and 15 are not significant.

The mathematical models developed from the DOE allow the optimization of photoprotective or multifunctional formulations containing the compositions of the invention, in a rational and predictable way, making the process of development of these formulations easier, faster and economical. With the help of the *software*, it is possible to calculate the quantities of each constituent of the compositions of the invention to obtain formulations with the desired values of parameters of photoprotective efficacy.

Table 6 presents one of the solutions generated by the *software*, containing the calculated amounts of each constituent of the invention to obtain photoprotective formulation with SPF values *in vitro,* UVAPFo and predefined critical wavelength (estimated) and the actual values obtained experimentally. The experimental results show an adequate correlation with the estimated parameters, proving that the method developed works.

**Table 6 - Solution generated by software, from the DOE, containing the calculated quantities of each constituent of the invention to obtain formulation with predefined (estimated) photoprotection parameters and the actual values, obtained experimentally.**

| **Concentration of constituents** | | | | | **Predefined photoprotection parameters** | | | **Experimentally obtained photoprotection parameters** | | |
|---|---|---|---|---|---|---|---|---|---|---|
| A | B | C | D | E | FPS *in vitro* | UVAPF 0 | Critical wavelength | FPS *in vivo*^{∗} | FPUVA^{∗} | Critical wavelength |
| 5 % | 11 % | 5 % | 2 % | 0 % | 39 | 18 | 378 nm | 34 | 14 | 378 nm |
| A- dispersion of titanium dioxide; B - dispersion of zinc oxide; C - acai oil; D - Kaolin; E: Sparclay^{®} Sgy. | | | | | | | | | | |
| Tests required by the National Health Surveillance Agency - ANVISA for the registration of sunscreen products: | | | | | | | | | | |
| ^{∗}According to ISO24443:2012 - *Determination of sunscreen UVA photoprotection in vitro.* | | | | | | | | | | |
| ^{∗∗}According to ISO24444:2010 Cosmetics - Sun protection test methods - *In vivo* determination of the sun protection factor. | | | | | | | | | | |

The method based on experimental design (DOE) developed in the present invention can be used employing other types of experimental design, other constituents that meet the requirements of the compositions of the invention and other responses of photoprotective efficacy, not restricted to the examples presented.

The photoprotective formulations of Examples #1, #2, #3 (Tables 7, 8 and 9) were prepared from compositions of the invention and tested for photoprotective efficacy *in vitro.*

**Table 7 - Example #1 - Preparation of photoprotective formulation FPS in vitro 13, UVAPFo 8, critical wavelength 379 nm and UVA/UVB ratio 0.753^{∗}.**

| **Phas e** | **Ingredient** | **INCI** | **Concentration** |
|---|---|---|---|
| 1 | JOP80MZCJ | Zinc oxide (and) Simmondsia chinensis (jojoba) seed oil (and) polyhydroxystearic acid (and) jojoba esters | 9.00% |
| 1 | Acai vegetable oil | Euterpe oleracea fruit oil (and) tocopherol | 4.00% |
| 1 | EMP50TEL | Titanium dioxide (and) ethyl macadamiate (and) silica (and) alumina (and) stearic acid (and) polyhydroxystearic acid | 3.00% |
| 1 | Lexfeel^{®} natural | Undecylenate Heptyl | 3.00% |
| 1 | Sensolene^{®} | Ethylhexyl olive oil | 2.00% |
| 1 | Kaolin | Kaolin | 2.00% |
| 1 | Sparclay^{®} Sgy | Kaolin | 0.50% |
| 1 | Tapioca^{®} Pure | Tapioca starch | 0.50% |
| 2 | Water | Aqua | 38.00% |
| 2 | Zemea^{®} | Propanediol | 6.00% |
| 2 | Olivem^{®} 1000 | Cetearyl olive oil (and) sorbitan olive tree | 5.00% |
| 2 | Oliwax^{®} LC | Cetyl palmitate (and) sorbitan palmitate (and) sorbitan olive tree | 3.00% |
| 2 | Olivem^{®} 300 | Olive oil peg-7 esters | 2.00% |
| 2 | Lexgard^{®} GMCY | Caprylate Glyceryl | 1.00% |
| 3 | Lemongrass hydrolato | Cymbopogon citratus flower water | 20.00% |
| 4 | Aromatic green tea extract | Camellia sinensis leaf extract | 1.00% |

| | | | |
|---|---|---|---|
| ^{∗}FPS *in vitro*, UVAPF₀, critical wavelength and UVA/UVB ratio determined in diffuse reflectance spectrophotometer with integrative sphere, model UV2000S (Labsphere^{®}). | | | |

**Table 8 - Example #2 - Preparation of sPF photoprotective formulation in vitro 60, UVAPFo 21, UVA/UVB ratio 0.725 and critical wavelength 378 nm^{∗}.**

| **Phase** | **Ingredient** | **INCI** | **Concentration** |
|---|---|---|---|
| 1 | JOP80MZCJ | Zinc oxide (and) Simmondsia chinensis (jojoba) seed oil (and) polyhydroxystearic acid (and) jojoba esters | 9.00% |
| 1 | Acai vegetable oil | Euterpe oleracea fruit oil (and) tocopherol | 8.00% |
| 1 | EMP50TEL | Titanium dioxide (and) ethyl macadamiate (and) silica (and) alumina (and) stearic acid (and) polyhydroxystearic acid | 7.00% |
| 1 | Lexfeel^{®} natural | Undecylenate Heptyl | 3.00% |
| 1 | Sensolene^{®} | Ethylhexyl olive oil | 2.00% |
| 1 | Kaolin | Kaolin | 2.00% |
| 1 | Sparclay^{®} Sgy | Kaolin | 0.50% |
| 1 | Tapioca^{®} Pure | Tapioca starch | 0.50% |
| 2 | Water | Aqua | 30.00% |
| 2 | Zemea^{®} | Propanediol | 6.00% |
| 2 | Olivem^{®} 1000 | Cetearyl olive oil (and) sorbitan olive tree | 5.00% |
| 2 | Oliwax^{®} LC | Cetyl palmitate (and) sorbitan palmitate (and) sorbitan olive tree | 3.00% |
| 2 | Olivem^{®} 300 | Olive oil peg-7 esters | 2.00% |
| 2 | Lexgard^{®} GMCY | Caprylate Glyceryl | 1.00% |
| 3 | Lemongrass hydrolato | Cymbopogon citratus flower water | 20.00% |
| 4 | Aromatic green tea extract | Camellia sinensis leaf extract | 1.00% |

| | | | |
|---|---|---|---|
| ^{∗}FPS *in vitro*, UVAPF₀, critical wavelength and UVA/UVB ratio determined in diffuse reflectance spectrophotometer with integrative sphere, model UV2000S (Labsphere^{®}). | | | |

**Table 9 - Example #3 - Preparation of photoprotective formulation FPS in vitro 91, UVAPFo 38, UVA/UVB ratio 0.786 and critical wavelength 379 nm^{∗}.**

| **Phase** | **Ingredient** | **INCI** | **Concentratio n** |
|---|---|---|---|
| 1 | JOP80MZCJ | Zinc oxide (and) Simmondsia chinensis (jojoba) seed oil (and) polyhydroxystearic acid (and) jojoba esters | 21.00% |
| 1 | Acai vegetable oil | Euterpe oleracea fruit oil (and) tocopherol | 4.00% |
| 1 | EMP50TEL | Titanium dioxide (and) ethyl macadamiate (and) silica (and) alumina (and) stearic acid (and) polyhydroxystearic acid | 7.00% |
| 1 | Lexfeel^{®} natural | Undecylenate Heptyl | 3.00% |
| 1 | Sensolene^{®} | Ethylhexyl olive oil | 2.00% |
| 1 | Kaolin | Kaolin | 2.00% |
| 1 | Sparclay^{®} Sgy | Kaolin | 0.50% |
| 1 | Tapioca^{®} Pure | Tapioca starch | 0.50% |
| 2 | Water | Aqua | 22.00% |
| 2 | Zemea^{®} | Propanediol | 6.00% |
| 2 | Olivem^{®} 1000 | Cetearyl olive oil (and) sorbitan olive tree | 5.00% |
| 2 | Oliwax^{®} LC | Cetyl palmitate (and) sorbitan palmitate (and) sorbitan olive tree | 3.00% |
| 2 | Olivem^{®} 300 | Olive oil peg-7 esters | 2.00% |
| 2 | Lexgard^{®} GMCY | Caprylate Glyceryl | 1.00% |
| 3 | Lemongrass hydrolato | Cymbopogon citratus flower water | 20.00% |
| 4 | Aromatic green tea extract | Camellia sinensis leaf extract | 1.00% |

| | | | |
|---|---|---|---|
| ^{∗}FPS *in vitro*, UVAPF₀, critical wavelength and UVA/UVB ratio determined in diffuse reflectance spectrophotometer with integrative sphere, model UV2000S (Labsphere^{®}). | | | |

In the production process of the photoprotective formulations of Examples#1, #2 and #3, phases 1 and 2 were prepared and heated separately up to 75 °C. After the emulsion reached 40 °C, phases 3 and 4 were added, in this order, under agitation.

The photoprotective formulation of Example #4 (Table 10) was prepared from compositions of the invention and tested for photoprotective efficacy *in vitro* and *in vivo.*

**Table 10 - Example #4 - Preparation of sPF photoprotective formulation in vivo 32, FPUVA14, UVA/UVB ratio 0.46 and critical wavelength 378 nm^{∗}.**

| **Phas e** | **Ingredient** | **INCI** | **Concentration** |
|---|---|---|---|
| 1 | Water | Aqua | 37.00 |
| 1 | Tapioca^{®} Pure | Tapioca Starch | 2.00 |
| 2 | Polyaquol^{™} -2W | Polyglyceryl-2-Stearate, Glyceryl Stearate, Stearyl Alcohol | 9.00 |
| 2 | Zemea^{®} | Propanediol | 6.00 |
| 2 | Olivem^{®} 300 | Olive Oil PEG-7 Esters | 2.00 |
| 2 | Lexgard^{®} GMCY | Glyceryl Caprylate | 1.00 |
| 3 | Acai vegetable oil | Euterpe oleracea fruit oil (and) tocopherol | 5.00 |
| 3 | Lexfeel^{®} natural | Heptyl Undecylenate | 3.00 |
| 3 | Sensolene^{®} | Ethylhexyl Olive Tree | 2.00 |
| 4 | Lemongrass Hydrolato | Cymbopogon citratus flower water | 10.00 |
| 4 | Kaolin | Kaolin | 2.00 |
| 4 | Sparclay SGY | Kaolin | 1.00 |
| 4 | Lime phytolipomas | Water(Aqua) (And) Calendula officinalis Flower Extract (And) Phospholipids (From Soybean Lecithin) (And) Tocopheryl Acetate | 3.00 |
| 4 | Aromatic Green Tea Extract | Camellia sinensis leaf extract | 1.00 |
| 5 | JOP80MZCJ | Zinc Oxide (And) Simmondsia Chinensis (Jojoba) Seed Oil (And) Polyhydroxystearic Acid (And) Jojoba Esters | 11.00 |
| 6 | EMP50TEL | Titanium dioxide (and) ethyl macadamiate (and) silica (and) alumina (and) stearic acid (and) polyhydroxystearic acid | 5.00 |
| ^{∗}According to ISO24443:2012 - Determination of sunscreen UVA photoprotection in vitro. | | | |
| ^{∗∗}According to ISO24444:2010 Cosmetics - Sun protection test methods - In vivo determination of the sun protection factor. | | | |

In the production process of the photoprotective formulation of Example#4, in a reactor, the water was heated to 40 °C and then the incorporation of tapioca began to be incorporated, gradually, under agitation. After total tapioca incorporation, agitation and heating were maintained until phase 1 reached 75 °C. The high shear system was activated for 5 minutes. In another reactor, phase 2 was heated to complete fusion (70-75 °C). Then, phase 2 was incorporated into phase 1 (75 °C), gradually under agitation. After the total incorporation of phase 2, the high shear system was activated for 10 minutes. In another reactor, phase 3 was heated to 75 °C and then incorporated into the mixture of phases 1 + 2, gradually, under agitation. After the total incorporation of phase 3, the high shear system was activated for 5 minutes. The mixture was kept under agitation during cooling. After 24 hours, in a reactor, under agitation, the kaolin, the Sparclay SGYthe calendula phytoliposomes and the aromatic extract of green tea, in that order, were dispersed in the hlemongrass idrode. Phase 4 was incorporated into the preformed cosmetic base, under agitation. After the total incorporation of phase 4, the high shear system was activated for 1 minute. Or JOP80MZCJ has been incorporated into the, gradually, and then the high shear system was triggered for 7 minutes. Or EMP50TEL has been incorporated into the, gradually and then the high shear system was activated for 13 minutes. Finally, agitation was maintained for 30 minutes.

The compositions of the present invention are useful for producing photoprotective and multifunctional formulations, intended for application on the skin. The constituents of the compositions of the present invention can be added at different stages of the production process of these formulations. For example: (1) these constituents may be added in different phases in the preparation of a formulation; (2) these constituents may be added in the form of a preformed mixture between them (*Blend*), in one of the phases in the preparation of a formulation; (3) these constituents may be incorporated in the form of a preformed mixture between them (*Blend*), on a previously prepared pharmaceutical or cosmetic basis.

The compositions of the present invention, when presented in the form of preformed mixture between its constituents (*Blend*), may contain other ingredients, for example, dispersing and stabilizing agents, but not limited to them. In addition to facilitating the incorporation of compositions in different pharmaceutical or cosmetic bases, these ingredients aim to improve other technological aspects.

The photoprotective or multifunctional formulations of the invention are products obtained by the association of said photoprotective compositions with other ingredients, for example, vehicles, tensoactives, emulsifiers, thickeners, gelling agents, emollients, wetting, stabilizers, preservatives, diluents, among others, which may be present depending on the type of pharmaceutical or cosmetic preparation formed, for example, emulsion, dispersion, paste, powder or rod.

The equipment used in the production of the compositions and formulations to which the invention refers are those known to be used in the production of pharmaceutical and cosmetic preparations, such as emulsions, dispersions, pastes, powders or rods.

In a preferred application, the ingredients of the compositions and formulations of the invention comply with the standards of natural and/or organic, approved by certifers such as Ecocert and Cosmos.

In specific applications, invention compositions and formulations can be effective not only for UVA, UVB and UVC, but also for other wavelength ranges, for example those of visible and infrared light.

An advantage of the present invention is that the method based on experimental design (*Design of Experiments* - DOE) developed allows the obtaining of formulations with the desired photoprotection parameters, from the compositions of the invention, rationally and predictably, which makes the development process easier, faster and more economical.

Finally, one more advantage of the present invention is that the photoprotective formulations optimized through the DOE present correlation *in vitro-in vivo* fps values.

It is important to note that the above description is intended solely to describe in an example of the particular embodiment of the invention in question. Therefore, it becomes clear that modifications, variations, and combinations of the above compositions remain within the scope of protection delimited by the attached claims.

## Claims

1. Photoprotective composition **CHARACTERIZED by** the fact that it contains: at least one clay, at least one oil and /or plant extract with photoprotective and /or antioxidant activity, and at least one inorganic sunscreen.

2. Photoprotective composition **CHARACTERIZED by** the fact that it contains: synergistically effective amounts of at least one clay, at least one oil and /or plant extract with photoprotective and /or antioxidant activity, and at least one inorganic sunscreen.

3. Photoprotective composition, according to one of claims 1 or 2, **CHARACTERIZED by** the fact that: said at least one clay is present in the composition in a concentration of 0.1 to 85% by weight, in the composition; said at least one oil and /or plant extract with photoprotective and/or antioxidant activity is present in the composition in a concentration of 0.2 to 80%, by weight; and said at least one inorganic sunscreen is present in the composition in a concentration of 0.5 to 75%, by weight.

4. Photoprotective composition according to one of claims 1 or 2 or 3, **CHARACTERIZED by** the fact that said at least one clay naturally contains one or more of the following compounds: silicon dioxide, aluminum oxide, iron oxide, potassium oxide, titanium dioxide, magnesium oxide, calcium oxide, phosphorus oxide, sodium oxide, zinc oxide, ferrous oxide, manganese oxide, lithium oxide, iron.

5. Photoprotective composition according to one of claims 1 or 2 or 3, **CHARACTERIZED by** the fact that said at least one clay naturally contains one or more of the following compounds: silicon dioxide, aluminum oxide, iron oxide, titanium dioxide, iron.

6. Photoprotective composition according to one of claims 1 or 2 or 3, **CHARACTERIZED by** the fact that said at least one clay is one or more from the following group: Tersil G, Tersil R, Tersil N, White Clay, Tersil CDR , Tersil CB, Tersil CP, Tersil COR, Tersil CBV, Tersil CGY, Cocoa Brown, Sparclay SW, Sparclay SR, Sparclay SBV, Sparclay SDR, Sparclay SOR, Sparclay SGY, Sparclay SP, Kaolin.

7. Photoprotective composition, according to one of claims 1 or 2 or 3, **CHARACTERIZED by** the fact that said at least one oil and /or plant extract naturally contains one or more compounds among: phenolic, nitrogenous, carotenoids, tocopherols and ascorbic acid.

8. Photoprotective composition according to one of claims 1 or 2 or 3, **CHARACTERIZED by** the fact that said at least one oil and/or plant extract is one or more from the following group: green tea extract, green coffee extract, extract calendula, yerba mate extract, ginger extract, rosemary essential oil, lemongrass essential oil, grapefruit essential oil, sweet orange essential oil, lavender essential oil, pitanga essential oil, verbena essential oil, açaí vegetable oil, karanja vegetable oil, rosehip vegetable oil.

9. Photoprotective composition according to one of claims 1 or 2 or 3, **CHARACTERIZED by** the fact that said at least inorganic sunscreen is one or more of the following group: titanium dioxide, zinc oxide and hydroxyapatite.

10. Photoprotective composition according to one of claims 1 or 2 or 3, **CHARACTERIZED by** the fact that said at least one inorganic sunscreen is one or more from the following group:
Titanium Dioxide, Hydrated Silica and Jojoba Esters;
Titanium Dioxide, Aluminum Hydroxide and Stearoyl Glutamic Acid;
Hydroxyapatite;
Titanium Dioxide, Ethyl Macadamiate, Silica, Alumina, Stearic Acid and Polyhydroxystearic Acid;
Butyloctyl Salicylate, Titanium Dioxide (nano) (40%), Triceteareth-4 Phosphate, Dimethicone Crosspolymer, Silica;
Butyloctyl Salicylate, TitaniumDioxide (nano) (23%), C12-15 Alkyl Benzoate, Ethylhexyl Methoxycrylene, Triceteareth-4 Phosphate, Dimethicone Crosspolymer, Silica; Zinc Oxide (60%), Butyloctyl Salicylate, Triceteareth-4 Phosphate, Triethoxycaprylylsilane;
Zinc Oxide (58%), Butyloctyl Salicylate, Ethylhexyl Methoxycrylene, Triceteareth-4 Phosphate, Triethoxycaprylylsilane;
Titanium Dioxide, Dimethicone;
Zinc Oxide, Simmondsia Chinensis Seed Oil, Polyhydroxystearic Acid, Jojoba Esters;
Titanium Dioxide, Stearoyl Glutamic Acid;
Titanium Dioxide, Alumina, Jojoba Esters;
Zinc Oxide nano; Zinc Oxide, Triethoxycaprylylsilane nano; Zinc Oxide and Triethoxycaprylylsilane;
Zinc Oxide;
Zinc Oxide, Stearoyl Glutamic Acid;
Zinc Oxide and Jojoba Esters.

11. Photoprotective composition according to one of claims 1 or 2 or 3, **CHARACTERIZED by** the fact that said at least one inorganic sunscreen is one or more from the following group: A15-TÍO2-S-NJE10, A1 K-TÍ02- ASG3 , Apalight, EMP50TEL, HalIBrite^{®}EZ-FLO TDX, HalIBrite^{®}EZ-FLO TDX Plus, HallBrite^{®}EZ-FLO ZDX, HalIBrite^{®}EZ-FLO ZDX Plus, HalIBrite^{®}T-97, JOP80MZCJ, TI02-IR300-SG3, TTO -NJE8, Z-COTE^{®}, Z-COTE^{®} HP1, Z-COTE^{®}LSA, ZnO-C, ZNO-C-ASG3J, ZnO-C-NJE3.

12. Photoprotective formulation **CHARACTERIZED by** including photoprotective composition, according to one of claims 1 or 2 or 3.

13. Photoprotective formulation, according to claim 12, **CHARACTERIZED by** being in the form of emulsion, dispersion, paste, powder or stick.

14. Multifunctional formulation CHARACTERIZED for including a photoprotective composition, according to one of claims 1 or 2 or 3.

15. Multifunctional formulation, according to claim 14, **CHARACTERIZED by** being in the form of emulsion, dispersion, paste, powder or stick.

16. Photoprotective formulation, according to claim 12, including photoprotective composition, according to one of claims 1 or 2 or 3, **CHARACTERIZED by** the fact that it contains:
Zinc oxide, Simmondsia chinensis (jojoba) seed oil, polyhydroxystearic acid, jojoba esters in a concentration of 9 to 21% by weight of the composition;
Euterpe oleracea fruit oil, tocopherol in a concentration of 4 to 8% by weight of the composition;
Titanium dioxide, ethyl macadamiate, silica, alumina, stearic acid, polyhydroxystearic acid in a concentration of 3 to 7% by weight of the composition;
Heptyl undecylenate in a concentration of 2 to 4% by weight of the composition;
Ethylhexyl olivate in a concentration of 1 to 3% by weight of the composition;
A first type of Kaolin in a concentration of 0.5 to 5% by weight of the composition;
A second type of Kaolin in a concentration of 0.5 to 5% by weight of the composition;
Starch tapioca in a concentration of 0.5 to 3% by weight of the composition;
Propanediol in a concentration of 4 to 8% by weight of the composition;
Cetearyl olivate and sorbitan olivate in a concentration of 3 to 7% by weight of the composition;
Cetyl palmitate, sorbitan palmitate, sorbitan olivate in a concentration of 1 to 5% by weight of the composition;
Olive oil peg-7 esters in a concentration of 1 to 3% by weight of the composition;
Glyceryl caprylate in a concentration of 0.5 to 1.5% by weight of the composition;
Cymbopogon citratus flower water in a concentration of 18 to 22% by weight of the composition;
Camellia sinensis leaf extract in a concentration of 0.5 to 1.5% by weight of the composition;
Aqua in sufficient concentration for 100% by weight of the composition.

17. Photoprotective formulation according to claim 12, including photoprotective composition according to one of claims 1 or 2 or 3, **characterized by** the fact that it contains:
Starch tapioca in a concentration of 0.5 to 3% by weight of the composition;
Polyglyceryl-2-stearate, glyceryl stearate, stearyl alcohol in a concentration of 6 to 12% by weight of the composition;
Propanediol from 4 to 8% by weight of the composition;
Olive oil peg-7 esters in a concentration of 1 to 3% by weight of the composition;
Glyceryl caprylate in a concentration of 0.5 to 2% by weight of the composition;
Euterpe oleracea fruit oil, tocopherol in a concentration of 3 to 7% by weight of the composition;
Heptyl undecylenate in a concentration of 2 to 4% by weight of the composition;
Ethylhexyl olivate in a concentration of 1 to 3% by weight of the composition;
Cymbopogon citratus flower water in a concentration of 8 to 16% by weight of the composition;
a first type of Kaolin in a concentration of 0.5 to 5% by weight of the composition;
a second type of Kaolin in a concentration of 0.5 to 5% by weight of the composition;
Water (Aqua), Calendula officinalis Flower Extract, Phospholipids (From Soybean Lecithin), Tocopheryl Acetate in a concentration of 1 to 5% by weight of the composition;
Camellia sinensis leaf extract in a concentration of 0.5 to 3% by weight of the composition;
Zinc Oxide, Simmondsia Chinensis (Jojoba) Seed Oil, Polyhydroxystearic Acid, Jojoba Esters in a concentration of 5 to 30% by weight of the composition;
Titanium dioxide, ethyl macadamiate, silica, alumina, stearic acid, polyhydroxystearic acid in a concentration of 2 to 10% by weight of the composition;
Aqua in sufficient concentration for 100% by weight of the composition.

18. Photoprotective formulation, according to claim 12, including photoprotective composition, according to one of claims 1 or 2 or 3, **CHARACTERIZED by** the fact that it contains:
Starch tapioca in a concentration of 2% by weight of the composition; Polyglyceryl-2-stearate, glyceryl stearate, stearyl alcohol at a concentration of 9% by weight of the composition;
Propanediol of 6% by weight of the composition;
Olive oil peg-7 esters in a concentration of 2% by weight of the composition;
Glyceryl caprylate at a concentration of 1% by weight of the composition;
Euterpe oleracea fruit oil (and) tocopherol in a concentration of 5% by weight of the composition;
Heptyl undecylenate at a concentration of 3% by weight of the composition;
Ethylhexyl olivate at a concentration of 2% by weight of the composition;
Cymbopogon citratus flower water at a concentration of 10% by weight of the composition;
A first type of Kaolin in a concentration of 2% by weight of the composition;
A second type of Kaolin at a concentration of 1% by weight of the composition;
Water (Aqua), Calendula officinalis Flower Extract, Phospholipids (From Soybean Lecithin), Tocopheryl Acetate in a concentration of 3% by weight of the composition;
Camellia sinensis leaf extract at a concentration of 1% by weight of the composition;
Zinc Oxide, Simmondsia Chinensis (Jojoba) Seed Oil, Polyhydroxystearic Acid, Jojoba Esters in a concentration of 11% by weight of composition;
Titanium dioxide, ethyl macadamiate, silica, alumina, stearic acid, polyhydroxystearic acid at a concentration of 5% by weight of the composition;
Aqua in a concentration of 37% by weight of the composition.

19. Method of preparing a photoprotective formulation according to one of claims 17 or 18, **CHARACTERIZED by** comprising the steps of:
Define the necessary quantities of each constituent of the photoprotective composition by experimental design;
Heat Aqua in a reactor to 40 ° C;
Incorporate Tapioca starch, under agitation, generating a first mixture;
Keep stirring and heating the first mixture to 75 ° C;
Submit the first mixture to a high shear system for five minutes;
Prepare a second mixture containing: Polyglyceryl-2-Stearate, Glyceryl Stearate, Stearyl Alcohol; Propanediol; Olive Oil PEG-7 Esters; Glyceryl Caprylate;
Heat the second mixture until complete melting, in a reactor, at a temperature between 70 to 75 ° C;
Incorporate the second mixture into the first mixture, little by little, under agitation, generating a third mixture;
Submit the third mixture to the high shear system for ten minutes;
Prepare a fourth mixture containing: Euterpe oleracea fruit oil (and) tocopherol; Heptyl Undecylenate; Ethylhexyl Olivate;
Heat the fourth mixture, in a reactor, to a temperature of 75 ° C;
Incorporate the fourth mixture into the third mixture, little by little, under agitation, generating a fifth mixture;
Submit the fifth mixture to the high shear system for five minutes; Cool the fifth mixture with stirring;
Wait for a period of time;
Prepare a sixth mixture, in a reactor, under agitation, dispersing the following in Cymbopogon citratus flower water: a first type of kaolin; a second type of kaolin; Water (Aqua), Calendula officinalis Flower Extract, Phospholipids (From Soybean Lecithin), Tocopheryl Acetate; Camellia sinensis leaf extract;
Incorporate the sixth mixture into the fifth mixture, under stirring, generating a seventh mixture;
Submit the seventh mixture to the high shear system for one minute;
Then, gradually add to the seventh mixture Zinc Oxide, Simmondsia Chinensis (Jojoba) Seed Oil, Polyhydroxystearic Acid, Jojoba Esters, generating an eighth mixture;
Submit the eighth mixture to the high shear system for seven minutes;
Gradually incorporate into the eighth mixture Titanium dioxide, ethyl macadamiate, silica, alumina, stearic acid, polyhydroxystearic acid, generating a ninth mixture;
Submit the ninth mixture to the high shear system for thirteen minutes; and
Stir the ninth mixture for 30 minutes.

20. Use of a photoprotective composition according to one of claims 1 to 11, **CHARACTERIZED by** protecting against ultraviolet radiation and / or combating photoaging.

21. Use of a photoprotective formulation according to one of claims 12, 13, 16, 17 or 18, **CHARACTERIZED by** protecting against ultraviolet radiation and / or combating photoaging.

22. Use of a multifunctional formulation according to one of claims 14 and 15, **CHARACTERIZED by** protecting from ultraviolet radiation and /or combat photoaging.

23. Use of a photoprotective formulation according to claim 14, **CHARACTERIZED by** containing other additives to serve as makeup, and may be in the form of emulsion, dispersion, paste, powder or stick.
